**Europäisches Patentamt**

**European Patent Office**

**Office européen des brevets**

(19)

(11) Veröffentlichungsnummer : **0 022 238**
**B1**

# (12) EUROPÄISCHE PATENTSCHRIFT

(45) Veröffentlichungstag der Patentschrift :
**01.09.82**

(21) Anmeldenummer : **80103724.3**

(22) Anmeldetag : **01.07.80**

(51) Int. Cl.³ : **C 07 C 49/185, C 07 C 45/37,**
**B 01 J 23/22, B 01 J 23/31,**
**B 01 J 23/68, B 01 J 23/89**

(54) Verfahren zur Herstellung von Methylglyoxal.

(30) Priorität : 07.07.79 DE 2927524

(43) Veröffentlichungstag der Anmeldung :
**14.01.81 (Patentblatt 81/02)**

(45) Bekanntmachung des Hinweises auf die Patenterteilung : **01.09.82 Patentblatt 82/35**

(84) Benannte Vertragsstaaten :
**BE DE FR GB NL**

(56) Entgegenhaltungen : **Keine**

(73) Patentinhaber : **HOECHST AKTIENGESELLSCHAFT**
**Postfach 80 03 20**
**D-6230 Frankfurt/Main 80 (DE)**

(72) Erfinder : **Baltes, Herbert, Dr.**
**Johannesallee 24**
**D-6230 Frankfurt am Main 80 (DE)**
Erfinder : **Leupold, Ernst Ingo, Dr.**
**Am Zäunefeld 15**
**D-6392 Neu-Anspach (DE)**

## Verfahren zur Herstellung von Methylglyoxal

Die Erfindung betrifft ein Verfahren zur Herstellung von Methylglyoxal durch heterogen-katalysierte Umsetzung von Glyzerin in der Gasphase.

Es ist bekannt, daß sich Glyzerin zweistufig über 1.3-Dihydroxyaceton zu Methylglyoxal umsetzen läßt (E.P. Zdrodovskaya und S.M. Zhdan-Pushkina, C.A. 55, 7511 c). Die Dehydrierung des Glyzerins zu 1.3-Dihydroxyaceton (Stufe 1) wird dabei mit Hilfe von Mikroorganismen (Acetobacter melanogenum) durchgeführt (s. auch : Houben-Weyl, Methoden der organischen Chemie, Band VII/12 a, S. 771 f). Das 1.3-Dihydroxyaceton läßt sich dann (Stufe 2) in Gegenwart eines sauren Katalysators zu Methylglyoxal umlagern (DE-PS 1 914 037).

Eine solche zweistufige Umsetzung erschwert eine kontinuierliche Reaktionsführung beträchtlich ; die bakterielle Dehydrierung (Stufe 1) erfordert außerdem stark verdünnte wäßrige Glyzerin-Lösungen und somit große Reaktionsvolumina. Diese Nachteile sowie lange Reaktionszeiten und komplizierte Aufarbeitungsschritte führen zu einer erheblichen Beeinträchtigung der wirtschaftlichen Nutzung dieses Zweistufen-Verfahrens.

Methylglyoxal wird industriell i. allg. durch katalytische Dehydrierung von Propylenglykol hergestellt. Hierbei treten als Neben- bzw. Folgeprodukte Formaldehyd, Kohlenoxide, Propionaldehyd, Hydroxypropanon, sowie hochsiedende, stabile Acetale bzw. Ketale auf, die durch die Reaktion der genannten Aldehyde bzw. Ketone mit nichtumgesetztem Propylenglykol entstehen (DE-PS 1 914 038, US-PS 2 339 346, US-PS 2 051 266).

Ziel der vorliegenden Erfindung ist daher in erster Linie die Schaffung eines kontinuierlichen Verfahrens zur Herstellung von Methylglyoxal, das die bisherigen Probleme überwindet.

Gegenstand der vorliegenden Erfindung ist ein kontinuierliches Verfahren zur Herstellung von Methylglyoxal, dadurch gekennzeichnet, daß man Glyzerin in der Gasphase über einen heterogen Dehydrierungskatalysator leitet.

Nach Houben-Weyl, Methoden der organischen Chemie, Band VII/2 a, S. 708 und Band VII/1, S. 235 f lagert sich Glyzerin an heterogenen Katalysatoren unter Wasserabspaltung leicht zu Acrolein um. Es war daher überraschend und nicht vorherzusehen, daß sich Glyzerin in der Gasphase einstufig an einem heterogenen Katalysator zu Methylglyoxal umsetzen läßt.

Das gasförmige Glyzerin wird entweder alleine oder — vorzugsweise — zusammen mit Wasserdampf und/oder einem Trägergas über den Katalysator geleitet. Als Trägergase kommen zum Beispiel Stickstoff und Edelgase in Frage, aber auch niedere gesättigte Kohlenwasserstoffe, wie Methan, Ethan oder Propan.

Es hat sich als vorteilhaft erwiesen, dem gasförmigen Glyzerin zusätzlich noch Sauerstoff oder ein sauerstoffhaltiges Gas, z.B. Luft, beizumischen. Falls man Luft verwendet, erfüllt diese gleichzeitig die Funktion des Trägergases.

Bei dem erfindungsgemäßen Verfahren setzt man pro Mol Glyzerin folgende Mengen an Zusatzstoffen ein :

Wasser : 0-15 Mol, vorzugsweise 0,1-8 Mol Sauerstoff oder sauerstoffhaltiges Gas : 0-8 Mol, vorzugsweise 0,5-5 Mol

Trägergas : 0-80 Mol, vorzugsweise 10-80 Mol, insbesondere 40-60 Mol

Auch außerhalb dieser Grenzen werden noch zufriedenstellende Ergebnisse erzielt.

Als Dehydrierungskatalysator setzt man im allgemeinen mindestens eines der folgenden Elemente ein, und zwar in metallischer Form oder aber als Verbindung : Vanadin, Molybdän, Wolfram, Kupfer, Silber, Zinn, Blei, Antimon, Wismut, Eisen. Vorzugsweise setzt man Vanadin, Molybdän, Kupfer, Silber, Zinn und/oder Antimon ein. Besonders bevorzugt sind Katalysatoren, die Vanadin und/oder Molybdän enthalten, insbesondere, wenn sie zusätzlich noch Zinn und/oder Silber enthalten.

Die genannten Elemente werden entweder in metallischer Form oder in Form ihrer Verbindungen, z.B. als Oxide, Nitrate, Acetate, Acetylacetonate, Oxalate, Citrate oder Halogenide, in die Reaktionszone eingebracht.

Jedoch zeigen auch andere Dehydrierungskatalysatoren eine katalytische Wirkung, insbesondere dann, wenn sie mindestens ein Element enthalten, das zur Bildung basischer oder amphoterer Oxide befähigt ist.

Es hat sich als vorteilhaft erwiesen, zur Aktivierung des Katalysators vor dem Einleiten des Glyzerins in die Reaktionszone ein oxydierendes Gas, insbesondere Sauerstoff oder Luft, oder ein reduzierendes Gas, insbesondere Wasserstoff oder mit Inertgas verdünnten Wasserstoff, bei Temperaturen von 100 bis 800 °C, insbesondere von 300 bis 600 °C über den Katalysator zu leiten. Die katalytisch aktiven Elemente werden vorzugsweise auf Trägermaterialien aufgezogen. Als Träger eignen sich vor allem Silicate, Aluminiumoxide, Aluminiumsilicate, Bimsstein oder Kohlen. Vorzugsweise verwendet man Silicate, Aluminiumoxide oder Aluminiumsilicate. Besonders vorteilhaft sind oberflächenarme Aluminiumsilicate mit einer BET-Oberfläche von weniger als 20 m²/g.

Die Gesamtmenge an katalytisch aktiven Elementen kann in weiten Grenzen schwanken. Im allgemeinen beträgt sie 0,01 bis 50 Gew.-%, vorzugsweise 0,1 bis 20 Gew.%, bezogen auf die Gesamtmasse des Trägerkatalysators. Die katalytisch aktiven Komponenten werden zweckmäßig in Form einer Lösung auf den Träger aufgebracht ; dann wird das Lösemittel abgedampft und der Katalysator getrocknet. Als Lösemittel verwendet man im allgemeinen Wasser, Salzsäure, Salpetersäure, Alkalilaugen oder wäßrige Ammoniaklösung, vorzugsweise Wasser.

Die aktiven Komponenten können jedoch auch ohne Träger eingesetzt werden.

Das erfindungsgemäße Verfahren wird im allgemeinen bei Temperaturen zwischen 100 und 600 °C, vorzugsweise zwischen 200 und 450 °C durchgeführt.

Die Verweilzeit liegt vorzugsweise zwischen 0,1 und 10 Sekunden, insbesondere jedoch zwischen 0,1 und 1 Sekunde. Auch außerhalb dieser Grenzen erhält man noch befriedigende Ergebnisse.

Das erfindungsgemäße Verfahren wird bevorzugt bei Normaldruck durchgeführt, jedoch können auch verminderte oder erhöhte Drucke angewendet werden (0,01 bis 100 bar).

Im einzelnen geht man so vor, daß das Glyzerin bzw. ein Gemisch aus Glyzerin und Wasser aus einer Dosiervorrichtung in eine Verdampfungszone und das entstandene Gas dann durch ein von außen beheiztes und mit dem Katalysator gefülltes Reaktionsrohr geleitet wird. In der Verdampfungszone erfolgt gegebenenfalls die Vermischung mit dem Trägergas und/oder dem Sauerstoff oder dem sauerstoffhaltigen Gas ; es hat sich als vorteilhaft erwiesen, diese Gase vor der Vermischung auf die Reaktionstemperatur aufzuheizen.

Die Reaktionsprodukte werden nach Verlassen des Reaktors zur Abtrennung der kondensierbaren Anteile gekühlt. Aus dem Kondensat läßt sich das Methylglyoxal nach üblichen Methoden, zum Beispiel gemäß DE-PS 1 914 038 durch Schnelldestillation oder gemäß US-PS 2 866 823 als Dimethylacetal isolieren.

Wenn ein Gemisch aus Glyzerin und Wasser eingesetzt wird, ist das Kondensat eine wäßrige Lösung, die für viele Einsatzgebiete auch direkt verwendet werden kann, z.B. zur Herstellung von Acetalen des Methylglyoxals (US-PS 2 421 559).

Methylglyoxal und die Acetale des Methylglyoxals eignen sich aufgrund ihrer hohen Reaktivität als Zwischenprodukte für die Herstellung zahlreicher chemischer Verbindungen, wie z.B. hochwirksamer Insektizide vom Allethrin-Typ (H.-J. Sanders und A.W. Taff, Ind. Eng. Chem. *46*, 414-426 (1954)).

Beispiel 1

12 ml/h einer 50 %igen wäßrigen Glyzerin-Lösung werden mit Hilfe einer Kolbenspritze über eine Verdampfungszone in einen senkrecht angeordneten Glasreaktor von 150 mm Länge und 20 mm Durchmesser eingeleitet. Dabei werden der Verdampfungszone gleichzeitig 56 Nl/h Stickstoff und 0,8 Nl/h Sauerstoff zugeführt, die beide vorher auf 350 °C aufgeheizt worden sind.

Der Reaktor wird von außen ebenfalls auf 350 °C geheizt und ist mit 15 ml eines Aluminiumsilicat-Katalysators gefüllt, welcher 4,6 Gew.% Vanadin und 5,4 Gew.-% Zinn enthält und eine BET-Oberfläche von ca. 1 m$^2$/g besitzt.

Zur Herstellung des Katalysators löst man 8,2 g Zinn-II-chlorid ($SnCl_2 \times 2H_2O$) und 8,5 g Ammoniumvanadat ($NH_4VO_3$) in 50 ml konzentrierter Salzsäure, tränkt 72 g Katalysatorträger mit dieser Lösung und dampft das Lösemittel auf dem Dampfbad ab. Der Katalysator wird anschließend bei 110 °C getrocknet und dann im Reaktor in einem Gasstrom aus 56 Nl/h Stickstoff und 3 Nl/h Sauerstoff drei Stunden lang auf 400 °C erhitzt.

Die Temperatur im Innern des Reaktors wird mit Hilfe eines Thermoelementes gemessen. Die Reaktionsprodukte werden in einer Kühlfalle bei − 70 °C kondensiert.

Nach einer Anlaufzeit von 1 h zur Einstellung konstanter Betriebsbedingungen wird der eigentliche Katalysatortest über einen Zeitraum von 2 h durchgeführt. Das Kondensat wird flüssigkeitschromatographisch analysiert.

Es werden als Ergebnis eines zweistündigen Versuches 60,3 mMol Methylglyoxal, entsprechend einer Selektivität von 76 % gefunden. Die Ausbeute an Acrolein, bezogen auf eingesetztes Glyzerin, ist kleiner 0,5 %.

Beispiel 2

Analog zu Beispiel 1 werden in die dort beschriebene Apparatur 6 ml/h Glyzerin, 3,7 Nl/h Sauerstoff und 56 Nl/h Stickstoff eingeleitet. Im Reaktor befinden sich 15 ml eines Aluminiumsilicat-Katalysators, der 5,0 Gew.-% Silber und 5,0 Gew.-% Vanadin auf einem Träger wie in Beispiel 1 enthält und auf 350 °C geheizt wird.

Als Ergebnis eines zweistündigen Versuches erhält man 96,8 mMol Methylglyoxal, entsprechend einer Selektivität von 81 %.

Beispiel 3

Analog zu Beispiel 1 werden in die dort beschriebene Apparatur 12 ml/h 50 %ige wäßrige Glyzerin-Lösung, 1,6 Nl/h Sauerstoff und 56 Nl/h Stickstoff eingeleitet. Im Reaktor befinden sich 15 ml eines Aluminiumsilicat-Katalysators, der 4,0 Gew.% Vanadin, 4,0 Gew.% Silber, 1,0 Gew.% Kupfer und 1,0 Gew.% Eisen auf einem Träger wie in Beispiel 1 enthält und auf 350 °C geheizt wird.

Als Ergebnis eines zweistündigen Versuches erhält man 80,8 mMol Methylglyoxal, entsprechend einer Selektivität von 82 %.

Beispiel 4

Analog zu Beispiel 1 werden in die dort beschriebene Apparatur 12 ml/h 50 %ige wäßrige Glyzerin-Lösung und 20 Nl/h Luft eingeleitet. Im Reaktor befinden sich 15 ml eines Aluminiumsilicat-Katalysators, der 4,0 Gew.-% Vanadin, 5,0 Gew.% Silber, 0,5 Gew.-% Molybdän und 0,5 Gew.-% Wismut auf einem Träger wie in Beispiel 1 enthält und auf 250 °C geheizt wird.

Als Ergebnis eines zweistündigen Versuches erhält man 70,7 mMol Methylglyoxal, entsprechend einer Selektivität von 69 %.

Beispiel 5

Analog zu Beispiel 1 werden in die dort beschriebene Apparatur 24 ml/h 50 %ige wäßrige Glyzerin-Lösung, 3,2 Nl/h Sauerstoff und 56 Nl/h Stickstoff eingeleitet. Im Reaktor befinden sich 15 ml eines Aluminiumsilicat-Katalysators, der 2,8 Gew.-% Vanadin, 6,8 Gew.-% Antimon, 0,3 Gew.-% Blei und 0,1 Gew.-% Wolfram auf einem Träger wie in Beispiel 1 enthält und auf 150 °C geheizt wird.

Als Ergebnis eines zweistündigen Versuches erhält man 129,2 mMol Methylglyoxal, entsprechend einer Selektivität von 55 %.


**Ansprüche**

1. Verfahren zur kontinuierlichen Herstellung von Methylglyoxal, dadurch gekennzeichnet, daß man Glyzerin in der Gasphase über einen heterogenen Dehydrierungskatalysator leitet.

2. Verfahren nach Anspruch 1, dadurch gekennzeichnet, daß der Katalysator mindestens eines der folgenden Elemente enthält : Vanadin, Molybdän, Wolfram, Kupfer, Silber, Zinn, Blei, Antimon, Wismut, Eisen.

3. Verfahren nach Anspruch 1, dadurch gekennzeichnet, daß der Katalysator mindestens eines der folgenden Elemente enthält : Vanadin, Molybdän, Kupfer, Silber, Zinn, Antimon.

4. Verfahren nach Anspruch 1, dadurch gekennzeichnet, daß der Katalysator Vanadin oder Molybdän oder beides enthält.

5. Verfahren nach Anspruch 4, dadurch gekennzeichnet, daß der Katalysator zusätzlich noch Zinn oder Silber oder beides enthält.

6. Verfahren nach einem der Ansprüche 2-5, dadurch gekennzeichnet, daß der Katalysator auf einem Träger mit einer BET-Oberfläche von weniger als 20 m²/g aufgebracht ist.

7. Verfahren nach einem der Ansprüche 1-6, dadurch gekennzeichnet, daß Glyzerin gemeinsam mit Wasser verdampft wird.

8. Verfahren nach einem der Ansprüche 1-7, dadurch gekennzeichnet, daß bei Temperaturen zwischen 100 und 600 °C gearbeitet wird.

9. Verfahren nach einem der Ansprüche 1-7, dadurch gekennzeichnet, daß bei Temperaturen zwischen 250 und 450 °C gearbeitet wird.

10. Verfahren nach einem der Ansprüche 1-9, dadurch gekennzeichnet, daß die Reaktion in Gegenwart von Sauerstoff oder einem Sauerstoff enthaltenden Gas durchgeführt wird.


**Claims**

1. Process for the continuous manufacture of methylglyoxal characterized by passing glycerol over a heterogeneous dehydrogenation catalyst in the gaseous phase.

2. Process according to claim 1, wherein the catalyst contains at least one of the following elements : vanadium, molybdenum, tungsten, copper, silver, tin, lead, antimony, bismuth, iron.

3. Process according to claim 1, wherein the catalyst contains at least one of the following elements : vanadium, molybdenum, copper, silver, tin, antimony.

4. Process according to claim 1, wherein the catalyst contains vanadium or molybdenum or both of them.

5. Process according to claim 4, wherein the catalyst additionally contains tin or silver or both of them.

6. Process according to any one of claims 2-5, wherein the catalyst is applied on a carrier having a BET-surface of less than 20 m²/g.

7. Process according to any one of claims 1 to 6, wherein glycerol is evaporated together with water.

8. Process according to claims 1 to 7, wherein the reaction temperatures are between 100 and 600 °C.

9. Process according to any one of claims 1 to 7, wherein the reaction temperatures are between 250 and 450 °C.

10. Process according to any one of claims 1 to 9, wherein the reaction is carried out in the presence of oxygen or an oxygen-containing gas.


**Revendications**

1. Procédé pour préparer en continu du méthylglyoxal, procédé caractérisé en ce qu'on fait passer du glycérol, en phase gazeuse, sur un catalyseur de déshydrogénation hétérogène.

2. Procédé selon la revendication 1, caractérisé en ce que le catalyseur contient au moins l'un des éléments suivants : vanadium, molybdène, tungstène, cuivre, argent, étain, plomb, antimoine, bismuth et fer.

3. Procédé selon la revendication 1, caractérisé en ce que le catalyseur contient au moins l'un des éléments suivants : vanadium, molybdène, cuivre, argent, étain et antimoine.

4. Procédé selon la revendication 1, caractérisé en ce que le catalyseur contient du vanadium ou du molybdène ou les deux à la fois.

5. Procédé selon la revendication 4, caractérisé en ce que le catalyseur contient en outre de l'étain ou de l'argent ou les deux à la fois.

6. Procédé selon l'une quelconque des revendications 2 à 5, caractérisé en ce que le catalyseur est appliqué sur support ayant une surface BET inférieure à 20 m²/g.

7. Procédé selon l'une quelconque des revendications 1 à 6, caractérisé en ce qu'on évapore le glycérol en même temps que de l'eau.

8. Procédé selon l'une quelconque des revendications 1 à 7, caractérisé en ce qu'on opère à des températures comprises entre 100 et 600 °C.

9. Procédé selon l'une quelconque des revendications 1 à 7, caractérisé en ce qu'on opère à des températures comprises entre 250 et 450 °C.

10. Procédé selon l'une quelconque des revendications 1 à 9, caractérisé en ce qu'on effectue la réaction en présence d'oxygène ou d'un gaz contenant de l'oxygène.